# EUROPEAN PATENT APPLICATION

(11) **EP 4 606 884 A1**
(43) Date of publication of application: **27.08.2025**
(21) Application number: 23886207.2
(22) Date of filing: 31.10.2023
(51) Int. Cl.: C12N 1/16, C12N 9/10, C12P 19/04, C12P 19/18, C12R 1/645

(54) **NOVEL AUREOBASIDIUM PULLULANS STRAIN FOR HIGH-EFFICIENCY PRODUCTION OF OLIGOSACCHARIDES AND USE THEREOF**

(30) Priority: 31.10.2022 KR 20220143089
(71) Applicant: CJ CheilJedang Corporation, Seoul 04560 (KR)
(72) Inventor: JEONG, Hye Yeon, Seoul 04560 (KR); KIM, Min Sung, Seoul 04560 (KR); KIM, Tae Keun, Seoul 04560 (KR)
(74) Representative: Meissner Bolte Partnerschaft mbB
(86) International application number: PCT/KR2023/017096
(87) International publication number: WO 2024/096511

(57) **Abstract**

Provided are an *Aureobasidium pullulans* CJFSKY2201 strain deposited under accession number KCCM13230P; a culture of the strain; use of the strain in producing oligosaccharides; a composition comprising the strain, a lysate thereof, a culture thereof, a concentrate thereof, or a dry product thereof; and a method of producing oligosaccharides, the method comprising the step of culturing the strain and the step of obtaining oligosaccharides from the culture.

## Description

### [Technical Field]

The present disclosure relates to an *Aureobasidium pullulans* CJFSKY2201 strain deposited under accession number KCCM13230P; a culture of the strain; use of the strain in producing oligosaccharides; a composition comprising the strain, a lysate thereof, a culture thereof, a concentrate thereof, or a dry product thereof; and a method of producing oligosaccharides, the method comprising the step of culturing the strain and the step of obtaining oligosaccharides from the culture.

### [Background Art]

Oligosaccharides play an important role in a variety of biological processes, and are used in dairy products, confectionery, beverages, etc. for the purpose of enhancing functionality, improving sweetness, and improving physical properties. Their functionality becomes known, leading to a growing interest in oligosaccharide synthesis. Currently, the best-selling oligosaccharide in Korea and abroad is fructooligosaccharide, which is widely used as a raw material for various health functional foods, and its sales volume is increasing every year. Starting with this, the overall demand for oligosaccharides is expected to continue to increase.

Oligosaccharides are found in natural foods such as tomatoes, bananas, onions, mushrooms, etc., but it is difficult to extract oligosaccharides directly from natural foods, and thus they are mostly synthesized. In addition, for research and practical application of oligosaccharides to medical and health functional foods, their large-scale synthesis is necessary, but synthesis of oligosaccharides is very difficult. Currently, enzymatic methods and whole-cell biocatalytic methods using microorganisms are used in the production of oligosaccharides, and chromatography and crystallization methods are known as existing methods of producing high-content fructooligosaccharide. However, both methods lack economic efficiency due to extremely low yields and difficulty in mass production (Korean Patent No. KR 10-1994-0005660 B1).

The demand for oligosaccharides has continued to increase, and facility investments have continued, but the development of strains for their production has not yet been carried out. However, further physical investment within the limited manufacturing space has become impossible, and the productivity improvement rate relative to the investment amount has also been insufficient.

Under this background, the present inventors have made efforts to increase the activity of a strain itself through the development of the strain itself for oligosaccharide production, and as a result, they have developed a strain that rapidly converts a sugar solution into oligosaccharides, thereby completing the present disclosure.

### [Disclosure]

### [Technical Problem]

A problem of the present disclosure is to provide an *Aureobasidium pullulans* CJFSKY2201 strain deposited under accession number KCCM13230P and a method of producing oligosaccharides therefrom.

### [Technical Solution]

An object of the present disclosure is to provide an *Aureobasidium pullulans* CJFSKY2201 strain deposited under accession number KCCM13230P.

Another object of the present disclosure is to provide a culture of the *Aureobasidium pullulans* CJFSKY2201 strain deposited under accession number KCCM13230P.

Still another object of the present disclosure is to provide a method of producing oligosaccharides, the method comprising the step of culturing the strain and the step of obtaining oligosaccharides from the culture.

### [Advantageous Effects]

In the present disclosure, it was confirmed that an *Aureobasidium pullulans* CJFSKY2201 strain deposited under accession number KCCM13230P has a high ability to synthesize oligosaccharides, and thus oligosaccharides may be effectively produced using the same, and oligosaccharides produced by the production method of the present disclosure may be usefully applied to the production of food compositions, feed compositions, etc.

### [Brief Description of the Drawing]

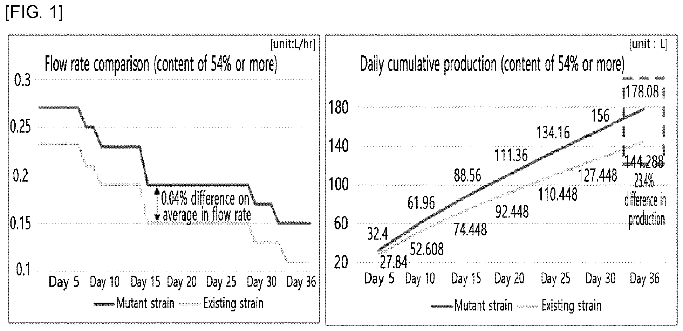

FIG. 1 shows graphs of flow rate and daily cumulative production for 36 days, resulting from oligosaccharide production reactions of an existing strain and a selected mutant strain.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

One aspect of the present disclosure provides an *Aureobasidium pullulans* CJFSKY2201 strain deposited under accession number KCCM13230P

As used herein, the *"Aureobasidium pullulans"* is a yeast-like fungus distributed in nature. *Aureobasidium pullulans* is known to be found in various environments such as forest soil, freshwater, and air, and on the surfaces of various plants, etc., and is known to have various enzymatic activities such as oligosaccharide synthase, siderophores, pullulan, etc.

The present inventors have developed a novel strain belonging to *Aureobasidium pullulans,* which was named *Aureobasidium pullulans* CJFSKY2201, and deposited under accession number KCCM13230P.

The "strain" of the present disclosure refers to a collection of individuals that originate from a single cell, propagate asexually, and have identical genetic traits. Within a species, there are many strains with different genetic traits. In the present disclosure, the strain refers to *Aureobasidium pullulans,* and specifically, may be *Aureobasidium pullulans* deposited under accession number KCCM13230P, but is not limited thereto.

The *Aureobasidium pullulans* CJFSKY2201 strain of the present disclosure may be an isolated strain, and a recombinant strain, wherein the recombination may be achieved by genetic modification. In addition, it may be a non-naturally occurring strain, or a mutant strain. In addition, the strain may comprise a cell, a dried cell, etc. of the *Aureobasidium pullulans* strain. The dried cell may be a dried cell, such as a spray-dried cell, a freeze-dried cell, a vacuum-dried cell, or a drum-dried cell, etc., but is not limited thereto.

In the present disclosure, in order to stably preserve the strain for a long period of time, the strain may be suspended in a storage solution which is prepared by mixing a predetermined amount of a glycerol component in water and stored at -70°C, or may be suspended in 10% sterilized skim milk and freeze-dried, but is not limited thereto. The strain may be in a long-term preservation form by various known methods.

The strain may have an increased ability to synthesize oligosaccharides, as compared to the wild type, and may have an increased activity of oligosaccharide synthase, and the oligosaccharide synthase may be fructosyltransferase, but is not limited thereto. Fructosyltransferase is an enzyme used to produce fructooligosaccharides using sugar or raw sugar as a raw material. It is known to have the function of hydrolyzing raw sugar or sugar and then binding one fructose molecule to the raw sugar or sugar molecule to synthesize oligosaccharides.

Still another aspect of the present disclosure provides a culture of the *Aureobasidium pullulans* CJFSKY2201 strain deposited under accession number KCCM13230P.

As used herein, the term "culturing (cultivation)" means growing a strain under appropriately artificially controlled environmental conditions. In the present disclosure, the "culture (cultured broth)" of the strain may be a culture broth comprising cells, or may be cells from which the culture supernatant is removed or which are concentrated. The composition of the culture may further comprise components that act synergistically on the growth of *Aureobasidium,* as well as common components that are needed for culturing *Aureobasidium,* and therefore, the composition may be easily selected by a person skilled in the art.

As for the media and other culture conditions used for culturing the strain of the present disclosure, any medium that is commonly used for culturing microorganisms of the genus *Aureobasidium* may be used without particular limitation. Specifically, the strain of the present disclosure may be cultured under aerobic or anaerobic conditions in a common medium comprising appropriate carbon sources, nitrogen sources, phosphorus sources, inorganic compounds, inorganic salts, amino acids, vitamins, and/or nucleic acids, etc., while controlling the temperature, pH, etc.

As the medium, a natural medium or a synthetic medium may be used. As the carbon sources of the medium, for example, glucose, sucrose, dextrin, glycerol, starch, etc. may be used. As the nitrogen sources, peptone, meat extract, yeast extract, dried yeast, soybeans, ammonium salts, nitrates and other organic or inorganic nitrogen-comprising compounds may be used, and as the phosphorus sources, potassium phosphate monobasic, potassium phosphate dibasic, or sodium-comprising salts corresponding thereto, etc. may be used, but are not limited to these components. As the inorganic compounds, sodium chloride, calcium chloride, iron chloride, magnesium sulfate, iron sulfate, manganese sulfate, calcium carbonate, etc. may be used, and as the inorganic salts, magnesium, manganese, calcium, iron, potassium, etc., may be used, but are not limited thereto.

In one embodiment of the present disclosure, the *Aureobasidium pullulans* strain was seed-cultured in a medium comprising sucrose and yeast extract at pH 5 to pH 6, and a main culture was performed at approximately pH 7 in a medium comprising sucrose, yeast extract, potassium phosphate dibasic, magnesium sulfate, and an antifoaming agent, etc., but is not limited thereto, and the components and input amounts of the respective materials may be appropriately adjusted.

Still another aspect of the present disclosure provides a bead on which the strain is immobilized. In the present disclosure, the bead is for immobilizing the strain, and is not particularly limited to its preparation method. The immobilization refers to an operation that confines cells to a certain space or part of an object while retaining their catalytic activities, and enables continuous reuse. In general, the characteristics of microorganisms should be considered when selecting an immobilization carrier and an immobilization method, and the immobilization method comprises an adsorption method, an entrapment method, etc. As the immobilization carriers, Ca-alginate, K-carrageenan, gelatin, chitosan, polyacrylamide gel, etc. may be used, but are not limited thereto. In one embodiment of the present disclosure, an alginate solution is mixed with a cell suspension, the mixture is filtered, and then a bead manufacturing tank is filled with the mixture, which is immersed in a calcium chloride (CaCl₂) solution to manufacture beads.

Still another aspect of the present disclosure provides a method of producing oligosaccharides, the method comprising the step of culturing the strain and the step of obtaining oligosaccharides from the culture.

As used herein, the terms "culturing (cultivation)" and "culture (cultured broth)" are as described above.

Oligosaccharides are either a sugar solution obtained through enzyme action for straight or branched binding of 10 or fewer sugar molecules, using sugar ingredients, or a liquid or powder obtained by filtering, refining, and concentrating the sugar solution. There are two types of food products: oligosaccharides and processed oligosaccharide products.

As used herein the term "oligosaccharide" refers to a saccharide having about 2 to about 10 sugar units, and its types comprise isomalto-oligosaccharide, fructo-oligosaccharide, galacto-oligosaccharide, xylo-oligosaccharide, gentio-oligosaccharide, malto-oligosaccharide, mixed oligosaccharides, etc.

The "fructo-oligosaccharide" of the present disclosure is a mixture of saccharides in which one or more fructose molecules, specifically, about 1 to about 5 fructose molecules, more specifically, about 1 to about 3 fructose molecules are bound to a fructose residue of a sugar, and comprises 1-kestose (GF2), nystose (GF3) and 1-F fructofuranosyl nystose (GF4), etc., but is not limited thereto. Fructo-oligosaccharides are mainly produced by enzymatic degradation of raw sugar or sugar as a raw material, but are not limited thereto. Fructo-oligosaccharides are used in various processed foods, and are widely used in the manufacture of rice cakes, castella, Korean traditional sweets, cakes, jellies, etc., but are not limited thereto.

The processed oligosaccharide product refers to a product processed by adding food or food additives to oligosaccharides, and refers to those comprising fructo-oligosaccharides, isomalto-oligosaccharides, galacto-oligosaccharides, xylo-oligosaccharides, or gentio-oligosaccharides in an amount of 10% or more, or malto-oligosaccharides in an amount of 40% or more.

In the present disclosure, a method of obtaining oligosaccharides using the strain may be a method widely known in the art, and may comprise a continuous process production method, a semi-batch production method, a batch production method, etc., but is not limited thereto. In addition, the method may be preferably a method of mixing and reacting the culture of the strain with sugar, and then obtaining oligosaccharides from the reaction product, or a method of isolating fructosyltransferase from the culture of the strain, mixing and reacting the isolated enzyme with sugar, and then obtaining oligosaccharides from the reaction product, but is not limited thereto. As used herein, "sugar (GF)" is also called sucrose or saccharose, and refers to a disaccharide in which one molecule of fructose and one molecule of glucose are linked by a glycosidic bond.

The step of mixing and reacting with sugar may be performed at a temperature range of 40°C to 60°C, but is not limited thereto, and the reaction time may be continued until the desired target material is produced.

The step of obtaining oligosaccharides may be performed by a common separation method known in the art. Such separation methods may comprise methods such as centrifugation, filtration, chromatography, crystallization, etc. For example, a supernatant obtained by removing biomass from a reaction product through low-speed centrifugation may be separated through ion exchange chromatography, but is not limited thereto. Alternatively, a process of separating and filtering the strain from the reaction product may be performed, and the target material may be recovered without a separate purification process. Alternatively, the recovery step may further comprise a purification process.

In the present disclosure, random mutations are induced by ultraviolet irradiation in the *Aureobasidium pullulans* strain used for oligosaccharide production, thereby developing a strain showing a high titer even under the same equipment and culture conditions, and thus making it possible to convert sugar into oligosaccharides faster in greater quantities than before. This not only leads to productivity improvement, but also enables response to urgent supplies. In addition, oligosaccharides may be produced in a shorter period of time than before, thereby drastically reducing the accompanying fixed costs such as fuel, manpower, etc., and oligosaccharides may be more stably produced.

Still another aspect of the present disclosure provides a composition comprising the *Aureobasidium pullulans* CJFSKY2201 strain deposited under accession number KCCM13230P, a lysate thereof, a culture thereof, a concentrate thereof, or a dry product thereof. More details regarding the above strain refer to the above-mentioned content.

The composition comprising the *Aureobasidium pullulans* CJFSKY2201 strain, the lysate thereof, the culture thereof, the concentrate thereof, or the dry product thereof may be utilized as a composition for producing oligosaccharides and utilized in producing food compositions or feed compositions.

The state of the strain may be a liquid state or a dry state, and the method for drying may comprise, but is not limited to, air drying, natural drying, spray drying, and freeze drying.

The composition may further comprise a cryoprotectant or an excipient. The cryoprotectant or excipient may be a non-naturally occurring substance or a naturally occurring substance, but is not limited thereto. In another specific embodiment, the cryoprotectant or excipient may be a substance that does not naturally come into contact with the *Aureobasidium pullulans* CJFSKY2201 strain, or a substance that is not naturally comprised simultaneously with the strain, but is not limited thereto. In still another specific embodiment, the composition may further comprise one or more cryoprotectants selected from the group consisting of glycerol, trehalose, maltodextrin, skim milk powder, and starch; and/or one or more excipients selected from the group consisting of glucose, dextrin, and skim milk. The cryoprotectant of the present disclosure may be comprised in an amount of 0.01% by weight to 20% by weight, 0.01% by weight to 10% by weight, based on the total weight of the composition. Specifically, the glycerol may be comprised in an amount of 5% by weight to 20% by weight, the trehalose may be comprised in an amount of 2% by weight to 10% by weight, the maltodextrin may be comprised in an amount of 2% by weight to 10% by weight, the skim milk powder may be comprised in an amount of 0.5% by weight to 2% by weight, and the starch may be comprised in an amount of 0.1% by weight to 1% by weight in the composition. In addition, the excipient may be comprised in an amount of 75% by weight to 95% by weight or 85% by weight to 95% by weight, based on the total weight of the composition.

Further, a method of preparing the composition comprising the *Aureobasidium pullulans* CJFSKY2201 strain, a lysate thereof, a culture thereof, a concentrate thereof, or a dry product thereof may comprise the step of mixing the *Aureobasidium pullulans* CJFSKY2201 strain, the lysate thereof, the culture thereof, the concentrate thereof, or the dry product thereof with an additive. The additive may be the above-mentioned cryoprotectant or excipient.

Still another aspect of the present disclosure provides a food composition comprising the oligosaccharide produced by the above production method.

As used herein, the term "oligosaccharides" is as described above.

As used herein, the term "food" may comprise meats, sausages, breads, chocolates, candies, snacks, confectionaries, pizzas, instant noodles, other noodles, gums, dairy products comprising ice creams, various kinds of soup, beverages, teas, drinks, alcoholic drinks, vitamin complexes, health functional foods, and health foods. Foods in the ordinary sense are all comprised.

The health functional food has the same meaning as "food for special health use (FoSHU)", and means a food having high pharmaceutical and medicinal effects, which is processed to efficiently exhibit bioregulatory functions in addition to nutrition supply. Here, the 'function' means obtaining effects useful for health applications, such as nutrient control or physiological actions on the structures and functions of the human body. The food of the present disclosure may be prepared by a method commonly used in the art, and may be prepared by adding raw materials and ingredients which are commonly added in the art during the above production. In addition, any formulation of the food may also be prepared without limitation, as long as it is acceptable as food.

The health food refers to a food having effects of actively maintaining or promoting health conditions, as compared to general foods, and a health supplement food refers to a food for supplementing health. If necessary, the health functional food, health food, and health supplement food may be interchangeably used with each other.

Specifically, the health functional food is a food prepared by adding the composition of the present disclosure to a food material such as beverages, teas, spices, gums, confectionery, etc., or prepared in a capsule, powder, or suspension form. The health functional food means that it has a specific effect on health when consumed, but unlike general drugs, the health functional food is free from side effects that may occur when a drug is taken for a long time, because foods are used as raw materials.

In this regard, the amount of the oligosaccharides comprised in the food is not particularly limited, but the oligosaccharides may be comprised in an amount of 0.01% by weight to 100% by weight, specifically, 1% by weight to 80% by weight, based on the total weight of the food composition.

The food composition may further comprise a physiologically acceptable carrier. The kind of carrier is not particularly limited. Any carrier may be used as long as it is commonly used in the art.

Further, the food composition may further comprise additional ingredients that are commonly used in food compositions so as to improve smell, taste, visuality, etc. For example, the food composition may comprise vitamins A, C, D, E, B1, B2, B6, B12, niacin, biotin, folate, pantothenic acid, etc. Further, the food composition may also comprise minerals such as zinc (Zn), iron (Fe), calcium (Ca), chromium (Cr), magnesium (Mg), manganese (Mn), copper (Cu), chromium (Cr), etc.; and amino acids such as lysine, tryptophan, cysteine, valine, etc.

Further, the food composition may comprise food additives, such as preservatives (potassium sorbate, sodium benzoate, salicylic acid, sodium dehydroacetate, etc.), disinfectants (bleaching powder, higher bleaching powder, sodium hypochlorite, etc.), antioxidants (butylhydroxyanisole (BHA), butylhydroxytoluene (BHT), etc.), colorants (tar color, etc.), color-developing agents (sodium nitrite, nitrous acid/sodium salt, etc.), bleaching agents (sodium sulfite), seasonings (monosodium glutamate (MSG), etc.), sweeteners (dulcin, cyclemate, saccharin, sodium, etc.), flavors (vanillin, lactones, etc.), swelling agents (alum, potassium D-bitartrate, etc.), fortifiers, emulsifiers, thickeners (adhesive pastes), film-forming agents, gum base agents, antifoaming agents, solvents, improvers, etc. The additives may be selected and used in an appropriate amount depending on the type of food.

Still another aspect of the present disclosure provides a feed composition comprising the oligosaccharides produced by the above production method.

As used herein, the term "oligosaccharides" is as described above.

As used herein, the term "feed" refers to any natural or artificial diet, a single meal, etc., or a component of the single meal, which an animal eats, ingests, and digests or which is suitable for eating, ingestion, and digestion.

The kind of feed is not particularly limited, and feeds commonly used in the art may be used. Non-limiting examples of the feed may comprise vegetable feeds such as grains, roots/fruits, food processing by-products, algae, fibers, pharmaceutical by-products, oils and fats, starches, gourds, and grain by-products; and animal feeds such as proteins, inorganic substances, oils and fats, minerals, oils and fats, single-cell proteins, animal planktons, or foods. These feeds may be used alone or in combination of two or more thereof.

The oligosaccharides which are comprised in the feed composition of the present disclosure may vary depending on the purpose of use and conditions of use of the feed. For example, the oligosaccharides may be comprised in an amount of 0.01% by weight to 100% by weight, more specifically, 1% by weight to 80% by weight, based on the total weight of the feed composition for livestock, but are not limited thereto.

Still another aspect of the present disclosure provides use of the *Aureobasidium pullulans* CJFSKY2201 strain deposited under accession number KCCM13230P in producing oligosaccharides.

### [Mode for Carrying Out the Invention]

Hereinafter, the present disclosure will be described in more detail with reference to the following exemplary embodiments. However, the following exemplary embodiments are only for illustrating the present disclosure, and the scope of the present disclosure is not intended to be limited thereby.

### Example 1. Method of Culturing Strain

In the present disclosure, seed culture of *Aureobasidium pullulans* strain was performed in a medium comprising sucrose and yeast extract at pH 5 to 6, and the average optical density (OD) was 10.

The main culture of the strain was performed in a medium comprising sucrose, yeast extract, potassium phosphate dibasic, magnesium sulfate, an antifoaming agent, etc. at approximately pH 7, and at this time, the average OD was 160.

### Example 2. Selection of Mutant Strain

### Example 2-1. Primary Selection

1 mL of the *Aureobasidium pullulans* strain culture was taken and diluted with 9 mL of 3 M sterile diluent (saline) and then dispensed into sterile plates (SPL Petri dish) for 10-fold dilution. Thereafter, the plates were irradiated with ultraviolet (UV) light for 20 seconds at a height in a clean bench for a predetermined period of time. After irradiation, 0.1 mL was taken from each plate and diluted 100-fold with 9.9 mL of 3 M sterile diluent (saline). Each 1 mL of the diluted solution was dispensed onto plates and cultured in a PDA (BD Difco) medium in a light-blocking manner using aluminum foil, and cultured at 28°C for 2 days to 3 days until the bacteria grew completely.

Groups were set according to the characteristics of the cultured strains, and 150 mL of the culture of each group was put in a 500 mL shaking flask (Shaking incubator, Lab tech) and pre-seeded at 220 RPM. 21 hours later, optical density (OD) and titer were measured to primarily select superior strains.

### Example 2-2. Secondary Selection

MCB 1 capsule (Thermo 1.8 mL) was purified, which was prepared by adding 0.9 mL of 40% glycerin to 0.9 mL of each culture of the mutant strains selected in Example 1-1. 20 strains were randomly selected therefrom, and 150 mL of the culture was put in a 500 mL shaking flask and pre-seeded at 240 RPM. 21 hours later, OD and titer were measured to select superior strains.

After purifying the MCB 1 capsule prepared with the selected mutant strains, the vials of strains showing high titers were purified, and 10 strains were randomly selected, and 150 mL of the culture was put in a 500 mL shaking flask and pre-seeded at 240 RPM. 21 hours later, OD and titer were measured to select superior strains.

### Example 2-3. Final Selection

Each 1 capsule of MCB prepared with the strains showing the high titers in Example 1-2 was purified, and then 10 strains were selected based on their characteristics and passaged in a slant medium. Thereafter, 150 mL of the culture was put in a 500 mL shaking flask and pre-seeded at 240 RPM. 21 hours later, OD and titer were measured to select superior strains.

**[Table 1]**

| Name of strain | OD | Titer | Improvement rate |
|---|---|---|---|
| WCB (control) | 24.4 | 105 | - |
| 4-1-7-1 | 23.7 | 130 | 24% increase in titer compared to control |
| 4-1-7-2 | 24 | 115.4 | 9% increase in titer compared to control |
| 4-1-7-3 | 25.5 | 96.7 | decrease in titer compared to control |
| 4-1-7-4 | 22.7 | 111.1 | 6% increase in titer compared to control |
| 4-1-7-5 | 20.6 | 101.7 | decrease in titer compared to control |
| 4-1-7-6 | 18.6 | 93.3 | decrease in titer compared to control |
| 4-1-7-7 | 23.3 | 105.9 | 1% increase in titer compared to control |
| 4-1-7-8 | 21.1 | 99.1 | decrease in titer compared to control |
| 4-1-7-9 | 19.4 | 99.7 | decrease in titer compared to control |
| 4-1-7-10 | 21.1 | 95 | decrease in titer compared to control |
| 4-1-7-11 | 22.6 | 89.3 | decrease in titer compared to control |
| 4-1-7-12 | 21.5 | 101.7 | decrease in titer compared to control |
| 4-1-7-13 | 20.5 | 96.7 | decrease in titer compared to control |
| 4-1-7-14 | 24.5 | 83.2 | decrease in titer compared to control |
| 4-1-7-15 | 17 | 94.9 | decrease in titer compared to control |
| 4-1-7-16 | 20.8 | 96.8 | decrease in titer compared to control |
| 4-1-7-17 | 21 | 75 | decrease in titer compared to control |
| 4-1-7-18 | 15.8 | 104.9 | decrease in titer compared to control |
| 4-1-7-19 | 18.5 | 91.6 | decrease in titer compared to control |
| 4-1-7-20 | 19.9 | 102.6 | decrease in titer compared to control |

As a result of the culture, as shown in Table 1, the strain 4-1-7-1 was confirmed to have an about 24% increase in the titer compared to the existing strain, and thus was finally selected as the best strain.

The selected strain was named *Aureobasidium pullulans* CJFSKY2201 and deposited with the Korean Culture Center of Microorganisms (KCCM), an international depository under the Budapest Treaty, on August 24, 2022, and was assigned the accession number KCCM13230P.

### Example 3. Pilot Reactor Experiment of Selected Mutant Strain

The mutant strain (4-1-7-1) finally selected and the existing strain were tested in a pilot reactor scaled down to a field-scale reactor. The reactor flow rate and productivity of the existing strain and the mutant strain were compared. The reactor jacket temperature was 49°C.

As a result of comparing the titers that directly affect the oligosaccharide productivity of the mutant strain (4-1-7-1) finally selected and the existing strain, as shown in Table 2 below, the existing strain showed a mean titer of 370 and the mutant strain showed a mean titer of 450, confirming that the mean titer was improved by about 20%, compared to the existing strain.

**[Table 2]**

| Section | Strain | OD | C.W | Mean titer | Improvement rate |
|---|---|---|---|---|---|
| Existing strain | WCB | 150 | 2.41 | 370 | - |
| Mutant strain | M4-1-7-1 | 165 | 2.54 | 450 | About 20% |

As a result of conducting the oligosaccharide production reaction for a total of 36 days, the mutant strain produced 178 L of oligosaccharide and the existing strain produced 144 L of oligosaccharide. As shown in Table 3 below, it was confirmed that the oligosaccharide productivity of the mutant strain was improved by about 23%, compared to the existing strain.

**[Table 3]**

| Section | Strain | Oligosaccharide content | Sucrose content | Average flow rate | Productivity improvement rate |
|---|---|---|---|---|---|
| Existing strain | WCB | 54% or more | 19.4% | 0.174 | - |
| Mutant strain | M4-1-7-1 | 54% or more | 18.4% | 0.213 | About 23% |

In addition, as shown in FIG. 1, the flow rate of the mutant strain increased by +0.04 on average compared to the existing strain, and the cumulative production of oligosaccharides for 36 days also increased by about 23%.

### Example 4. Field Reactor Experiment of Selected Mutant Strain

The mutant strain finally selected and the existing strain were compared using a field reactor. Other culture conditions were the same as those in the pilot reactor experiment, and the flow rate and production volume in the field reactor were compared. As a result, it was confirmed that the culture patterns, such as titer, pH, DO, OD, and C/W, etc., were almost similar to those in the pilot reactor experiment. In addition, as a result of the field culture, it was confirmed that the mutant strain showed an about 10% improvement in oligosaccharide productivity compared to the existing strain, as shown in Table 4 below.

**[Table 4]**

| Section | Strain | Oligosaccharide content | Sucrose content | Average flow rate | Productivity improvement rate |
|---|---|---|---|---|---|
| Existing strain | WCB | 54% or more | 20.4% | 470 | - |
| Mutant strain | M4-1-7-1 | 54% or more | 18.9% | 516 | About 10% |

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Aureobasidium pullulans CJFSKY2201 | Accession number given by the
INTERNATIONAL DEPOSITARY AUTHORITY:
KCCM13230P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on August. 24. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: August. 24. 2022.

¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel. 02-391-0950, 396-0950 Fax. 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. An *Aureobasidium pullulans* CJFSKY2201 strain deposited under accession number KCCM13230P.

2. The strain of claim 1, wherein the strain has an increased ability to synthesize oligosaccharides, as compared to the wild-type.

3. A culture of an *Aureobasidium pullulans* CJFSKY2201 strain deposited under accession number KCCM13230P.

4. A method of producing oligosaccharides, the method comprising
(a) the step of culturing the strain of claim 1; and
(b) the step of obtaining oligosaccharides from the culture.

5. The method of claim 4, wherein the step (b) is mixing and reacting the culture with sugar, and then obtaining oligosaccharides from the reaction product.

6. The method of claim 4, wherein the step (b) is separating a fructosyltransferase enzyme from the culture, and mixing and reacting the separated enzyme with sugar, and then obtaining oligosaccharides from the reaction product.

7. The method of any one of claims 4 to 6, wherein the oligosaccharides are fructo-oligosaccharides.

8. The method of claim 7, wherein the fructo-oligosaccharides comprise 1-kestose, nystose, and 1-F fructofuranosyl nystose.

9. The method of any one of claims 5 and 6, wherein the reacting step is performed at a temperature range of 40°C to 60°C.

10. A composition comprising an *Aureobasidium pullulans* CJFSKY2201 strain deposited under accession number KCCM13230P, a lysate thereof, a culture thereof, a concentrate thereof, or a dry product thereof.

11. Use of an *Aureobasidium pullulans* CJFSKY2201 strain deposited under accession number KCCM13230P in producing oligosaccharides.
